# EUROPÄISCHE PATENTSCHRIFT

(11) **EP 1 401 797 B1**
(45) Veröffentlichungstag und Bekanntmachung des Hinweises auf die Patenterteilung: **09.08.2006**
(21) Anmeldenummer: 02743180.8
(22) Anmeldetag: 12.06.2002
(51) Int. Cl.: C07C 29/50, C07C 45/34, C07C 51/25, C07B 41/00, C07C 35/205, C07C 49/413

(54) **VERFAHREN ZUR HERSTELLUNG GESÄTTIGTER ALKOHOLE, KETONE, ALDEHYDE UND CARBONSÄUREN**
METHOD FOR PRODUCING SATURATED ALCOHOLS, KETONES, ALDEHYDES AND CARBOXYLIC ACIDS
PROCEDE DE PRODUCTION D'ALCOOLS, DE CETONES, D'ALDEHYDES ET D'ACIDES CARBOXYLIQUES SATURES

(30) Priorität: 02.07.2001 DE 10131522
(43) Veröffentlichungstag der Anmeldung: 31.03.2004
(73) Patentinhaber: Degussa AG, 40474 Düsseldorf (DE)
(72) Erfinder: KÜHNLE, Adolf, 45770 Marl (DE); JOST, Carsten, 45770 Marl (DE); SHELDON, Roger Arthur, NL-2281 VA Rijswijk (NL); CHATEL, Sandrine M. M., NL-2641 ZV Pijnacker (NL); ARENDS, Isabella W.C.E., NL-2548 SL's Gravenhage (NL)
(74) Vertreter: Olbricht, Gerhard
(86) Internationale Anmeldenummer: PCT/EP2002/006411
(87) Internationale Veröffentlichungsnummer: WO 2003/004447

(56) Entgegenhaltungen:
- EP-A- 0 858 835
- US-A- 5 030 739
- Y. ISHII: "A novel catalysis of N-hydroxyphthalimide in the oxidation of organic substrates by molecular oxygen" JOURNAL OF ORGANIC CHEMISTRY, Bd. 60, Nr. 13, 1995, Seiten 3934-3935, XP000512054 EASTON US in der Anmeldung erwähnt
- A. HORINAKA: "Photosensitized oxygenation of unconjugated cyclic dienes" BULLETIN OF THE CHEMICAL SOCIETY OF JAPAN, Bd. 48, Nr. 7, 1975, Seiten 2095-2098, XP002253156 TOKYO JP

## Beschreibung

Die Erfindung betrifft ein Verfahren zur Herstellung von gesättigten Alkoholen, Ketonen, Aldehyden bzw. Carbonsäuren durch katalytische Oxidation ungesättigter Kohlenwasserstoffe und anschliessender Hydrierung der Oxidationsprodukte.

Die Oxidation von Kohlenwasserstoffen ist eine wichtige Reaktion in der industriellen organischen Chemie. Für diese Oxidationen können Verbindungen wie KMnO₄, CrO₃ oder HNO₃ als Oxidationsmittel eingesetzt werden. Diese weisen aber einerseits den Nachteil eines relativ hohen Preises auf, andererseits bringt ihre Verwendung unerwünschte Nebenprodukte mit sich, die zu Entsorgungsproblemen und ökologischer Belastung führen können.

Bevorzugt werden deshalb Oxidationsmittel auf der Basis von Peroxiden oder N₂O verwendet. Das preiswerteste Oxidationsmittel stellt aber molekularer Sauerstoff, entweder in reiner Form oder als Luftsauerstoff, dar. Sauerstoff für sich allein genommen ist allerdings für die Oxidation von Kohlenwasserstoffen zumeist nicht geeignet, da die Reaktivität des O₂₋Moleküls, das in der energetisch günstigen Triplett-Form vorliegt, nicht ausreichend ist.

Durch den Einsatz von Redoxmetallkatalysatoren ist es möglich, molekularen Sauerstoff für die Oxidation von organischen Verbindungen zu nutzen. Eine ganze Reihe industrieller Verfahren basieren auf der metallkatalysierten Autoxidation von Kohlenwasserstoffen. So erfolgt z. B. die Oxidation von Cyclohexan mit O₂ zu Cyclohexanol bzw. Cyclohexanon unter Verwendung von Mangan- oder Kobaltsalzen ("Industrielle Organische Chemie" 1994, 260, VCH Verlagsgesellschaft mbH Weinheim). Diese industriellen Verfahren basieren auf einem Radikalkettenmechanismus. Das Diradikal Sauerstoff reagiert dabei mit einem Kohlenwasserstoffradikal unter Bildung eines Peroxyradikals und anschliessender Kettenfortpflanzung durch Abstraktion eines H-Atoms an einem weiteren Kohlenwasserstoff. Neben Metallsalzen können aber auch organische Moleküle als Radikalstarter fungieren.

Nachteilig bei diesen Verfahren ist, dass die Selektivität mit steigendem Umsatz sehr stark sinkt und deshalb die Verfahren auf niedrigem Umsatzniveau gefahren werden müssen. So wird beispielweise die Oxidation von Cyclohexan zu Cyclohexanol / Cyclohexanon bei einem Umsatz von 10 bis 12 % durchgeführt, damit die Selektivität 80 bis 85 % beträgt ("Industrielle Organische Chemie" 1994, 261, VCH Verlagsgesellschaft mbH Weinheim). In einem weiteren wichtigen industriellen Autoxidationsprozess, der Cumol-Oxidation zu Cumolhydroperoxid, beträgt der Umsatz ca. 30 % bei einer Cumolhydroperoxid-Selektivität von ca. 90 % ("Industrielle Organische Chemie" 1994, 383, VCH Verlagsgesellschaft mbH Weinheim).

Eine Alternative zu Metallkatalysatoren stellt die Verwendung von Katalysatorsystemen wie z. B. N-Hydroxyphthalimid (NHPI) dar. Jedoch ist die Reaktionsgeschwindigkeit bei den vorgestellten Verfahren trotz der hohen Menge an Katalysator (bis zu äquimolaren Verhältnissen gegenüber dem Substrat) nicht befriedigend (J. Mol. Catalysis A. 1997, *117*, 123 - 137). US 5 030 739 beschreibt die Verwendung von N-Hydroxydicarbonsäureimiden zur allylischen Oxidation von Isoprenderivaten zu den entsprechenden Acroleinverbindungen.

Im allgemeinen werden Mengen an Katalysator von mindestens 10 mol% im Verhältnis zum Substrat eingesetzt, wobei höhere Mengen an Katalysator zur Steigerung der Reaktionsgeschwindigkeit verwendet werden (*J. Org. Chem.* 1995, *60,* 3934-3935).

Eine Weiterentwicklung des Systems stellt die Verwendung von Co-Katalysatoren dar. Als Co-Katalysatoren können Metallverbindungen, insbesondere Schwermetallsalze, Enzyme oder starke Brönsted-Säuren verwendet werden. So zeigten Ishii et al., dass NHPI in Verbindung mit Metallsalzen als Co-Katalysator Vorteile gegenüber der Oxidation mit NHPI aber ohne Metallsalz aufweisen können (z. B. EP 0878234, EP 0864555, EP 0878458, EP 0858835, JP 11180913, J. Mol. Catalysis A. 1997,117, 123 - 137). Nachteilig an diesem System ist aber, neben dem unerwünschten Schwermetallgehalt auch hier die hohe Menge an verwendetem NHPI. Um eine zufriedenstellende Reaktionsgeschwindigkeit zu gewährleisten, müssen mindestens 10 mol% an Katalysator verwendet werden. Weiterhin nachteilig ist, dass die eingesetzten Redoxmetalle teilweise weitergehende Reaktionen der Produkte katalysieren und so die Selektivität der Reaktion vermindern.

Es sind auch Verfahren bekannt geworden, die nur einen Katalysator ohne Co-Katalysator verwenden. Diese sind jedoch auf die Oxidation von besonders aktivierten Substraten wie Ether, Ester oder Isoprenderivate beschränkt.

Eine weitere Verfahrensvariante stellt die Verwendung von NHPI in Verbindung mit Alkoholen oder Aldehyden dar (Chem. Commun. 1999, 727 - 728, Tetrahedron Letters 1999, *40*, 2165 - 2168, Chem. Commun. 1997, 447 - 448). Nachteilig an diesen Verfahren ist die Bildung von Koppelprodukten und das verwendete hohe Katalysator-Substrat-Verhältnis (10 mol%).

In DE 19723890 wird ein Oxidationssystem, bestehend aus einem organischen Katalysator (3-Amino-NHPI) und dem Redoxenzym Laccase, für die Herstellung aromatischer und heteroaromatischer Aldehyde und Ketone beschrieben. Auch hier ist die eingesetzte Menge an Katalysator sehr hoch. Zudem weist dieses Verfahren durch die Verwendung eines Enzyms ein kompliziertes Reaktionssystem mit einem biologisch notwendigen Puffersystem auf, das die breite Anwendbarkeit dieses Systems einschränkt. Eine Verwendung von NHPI zur Oxidation von Olefinen, verbunden mit anschliessender Hydrierung, ist bis jetzt noch nicht beschrieben worden.

Bull. Chem. Soc. Japan, Band 48, Nr. 7, 2095-2098 beschreibt die Herstellung von Cyclododecanol durch photochemische Oxidation von 1,5,9-Cyclododecatriene mit O₂ und anschließende Hydrierung.

Die Aufgabe der vorliegenden Erfindung bestand darin, ungesättige Kohlenwasserstoffe durch Oxidation in gesättigte Alkohole, Aldehyde oder Ketone selektiv zu überführen. Hierzu sollten insbesondere keine Schwermetallsalze wie z. B. Kobaltacetat verwendet werden.

Überraschenderweise wurde gefunden, dass Verbindungen des Typs 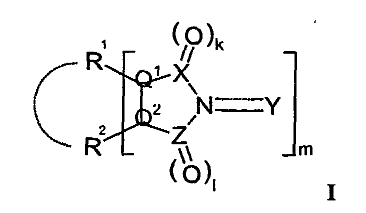 zur allylischen Oxidation von ungesättigten Kohlenwasserstoffen eingesetzt werden können und dass eine anschliessende Hydrierung des Oxidationsproduktes ausreicht, um die Anzahl der bei der Oxidation gebildeten Isomere zu reduzieren und somit mit hoher Selektivität gesättigte mit Sauerstoff funktionalisierte Produkte hergestellt werden können.

Gegenstand der vorliegenden Erfindung ist daher ein Verfahren zur Herstellung von gesättigten Alkoholen, Ketonen, Aldehyden, Ketonen und Carbonsäuren, wobei als Ausgangsverbindung ein ungesättigter Kohlenwasserstoff eingesetzt wird, der mit einem sauerstoffhaltigen Gas unter Verwendung eines Katalysators und Anwesenheit eines Radikalstarters zu einem Oxidationsprodukt oxidiert wird, und als Katalysator eine Verbindung der Formel **I** 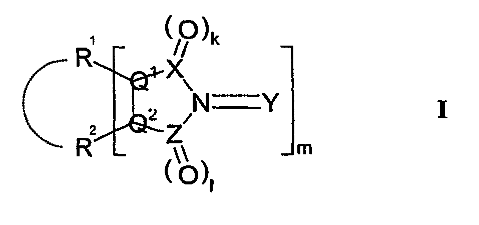 mit R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können,
- Q¹, Q²: = gleich oder ungleich, C, CH oder N,
- X, Z: = C, S oder CH₂,
- Y: = O oder OH,
- k: = 0, 1 oder 2,
- l: = 0, 1 oder 2 und
- m: =1 bis 100
eingesetzt wird,
dadurch gekennzeichnet,
dass die Oxidationsprodukte durch eine anschliessende Hydrierung reduziert werden und gesättigte Alkohole, Ketone, Aldehyde und/oder Carbonsäuren gebildet werden.

Das erfindungsgemäße Verfahren hat den Vorteil, dass auch ohne Co-Katalysator, Schwermetalle oder starke Säuren mittels Oxidation ungesättigte Kohlenwasserstoffe zu Alkoholen, Aldehyden und Ketonen umgesetzt werden können. Ein weiterer Vorteil der Kombination von Oxidation mit dem oben genannte Katalysator und anschließender Hydrierung des Oxidationsprodukt liegt darin, dass die Anzahl der bei der Oxidation gebildeten Isomeren reduziert wird und somit mit hoher Selektivität gesättigte mit Sauerstoff funktionalisierte Produkte erhalten werden können. Dies ist erst durch die erfindungsgemäße Kombination von Oxidation und Hydrierung möglich. Bei Oxidationen gemäß dem Stand der Technik ohne anschließende Hydrierung wird ein Isomeren-Gemisch erhalten, welches nur mühsam aufbereitet werden kann.

Das erfindungsgemäße Verfahren zur Herstellung von gesättigten Alkoholen, Ketonen, Aldehyden, Ketonen und Carbonsäuren zeichnet sich dadurch aus, dass als Ausgangsverbindung ein ungesättigter Kohlenwasserstoff eingesetzt wird, der mit einem sauerstoffhaltigen Gas unter Verwendung eines Katalysators und Anwesenheit eines Radikalstarters zunächst oxidiert wird, und als Katalysator eine Verbindung der Formel **I** 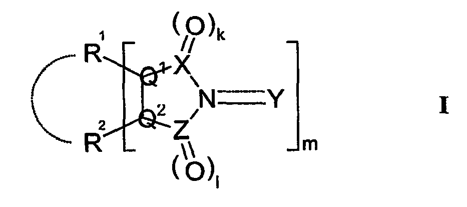 mit R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können,
- Q¹, Q²: = gleich oder ungleich, C, CH oder N,
- X, Z: = C, S oder CH₂,
- Y: = O oder OH,
- k: = 0, 1 oder 2,
- l: = 0, 1 oder 2 und
- m: = 1 bis 100
eingesetzt wird,
und die Oxidationsprodukte durch eine anschliessende Hydrierung reduziert werden, wodurch gesättigte Alkohole, Ketone, Aldehyde und/oder Carbonsäuren gebildet werden.

Beispiele für Verbindungen der Formel **I** sind N-Hydroxyphthalimid, 4-Amino-N-Hydroxyphthalimid, 3-Amino-N-Hydroxyphthalimid, Tetrabromo-N-Hydroxyphthalimid, Tetrachloro-N-Hydroxyphthalimid, N-Hydroxyhetimid, N-Hydroxyhimimid, N-Hydroxytrimellitimid, N-Hydroxy-benzol-1,2,4-tricarbonsäureimid, N,N'-Dihydroxypyromellitsäurediimid, N,N'-Dihydroxy-benzophenon-3,3',4,4'tetracarbonsäurediimid, N-Hydroxymaleimid, N-Hydroxy-Pyridin-2,3-dicarbonsäureimid, N-Hydroxysuccinimid, N-Hydroxyweinsäureimid" N-Hydroxy-5-norbonen-2,3-dicarbonsäureimid, exo-N-Hydroxy-7-oxabicyclo[2.2.1]-hept-5-en-2,3-dicarboximid, N-Hydroxy-cis-cyclohexan-1,2-dicarboximid, N-Hydroxy-cis-4-cyclohexen-1,2-dicarbonsäureimid, N-Hydroxynaphthalsäureimid-NatriumSalz oder N-Hydroxy-o-benzoldisulfonimide, Hydantoin und Derivate von Hydantoinen sowie N-Hydroxysaccharin.

Im erfindungsgemässen Verfahren werden keine Metallverbindungen oder Enzyme als Co-Katalysator verwendet. Das Verfahren kann in Masse durchgeführt werden, bevorzugt wird das Verfahren aber in organischen Lösungsmitteln unter Abwesenheit von starken Säuren durchgeführt, die Verwendung einer wässrigen Lösung, deren pH-Wert sich im schwach sauren bis basischen Bereich bewegen kann, ist ebenfalls möglich.

Die Oxidation wird vorzugsweise so durchgeführt, dass das molare Verhältnis des Katalysators zu dem zu oxidierenden Kohlenwasserstoff, also den gesättigten und/oder ungesättigten zyklischen Kohlenwasserstoffen, von 10⁻⁸ bis 1, bevorzugt von 10⁻⁷ bis 0,5, ganz besonders bevorzugt von 10⁻⁶ bis 0,2 und in einer speziellen Ausführungsform von 10⁻³ bis 0,1 beträgt.

In speziellen Ausführungsformen des erfindungsgemässen Verfahrens können auch Derivate bzw. Spezialfälle von Verbindungen der Formel **I** eingesetzt werden.

Bevorzugt werden Katalysatoren der Formel **II,** d. h. Verbindungen gemäss Formel **I** mit Q = C und m = 1 eingesetzt, 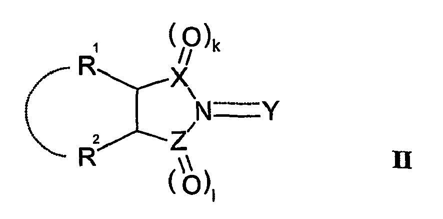 wobei R¹, R², X, Y, Z, k und l die für Verbindungen der Formel I definierten Bedeutungen haben.

Ganz besonders bevorzugt werden Katalysatoren der Formel III 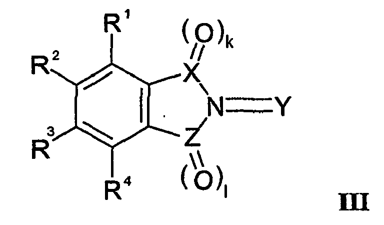 mit R¹, R², R³ und R⁴ = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹, R², R³ und R⁴ identische oder unterschiedliche Reste bezeichnen können,
- X, Z: = C, S und/oder CH₂,
- Y: = O oder OH,
- k: = 0, 1 oder 2 und
- l: = 0, 1 oder 2, wobei k und 1 nicht gleichzeitig den Wert 0 einnehmen dürfen eingesetzt.

Die erfindungsgemäße Oxidation erfolgt vorzugsweise in der Flüssigphase bei einer Temperatur von 0 bis 300 °C, bevorzugt bei einer Temperatur von 50 bis 200 °C. Dabei kann sowohl ein Lösemittel bzw. Lösemittelgemisch als auch die zu oxidierende Verbindung selbst als Lösemittel verwendet werden.

Die zu oxidierenden Verbindungen gehören in der Regel zu der Gruppe der Kohlenwasserstoffe. Mit Hilfe des erfindungsgemässen Verfahrens können eine Vielzahl ungesättigter organischer Verbindungen wie lineare und verzweigte Alkene, Diene und Triene mit einer Kohlenstoffzahl von 3 bis 25 sowie substituierte und unsubstituierte Cycloalkene, substituierte und unsubstituierte zyklische Diene sowie substituierte und unsubstituierte Triene mit einer Ringzahl von 5 bis 25 oxidiert und anschliessend hydriert werden, wobei in hoher Selektivität die entsprechenden Alkohole, Ketone, Aldehyde und/oder Carbonsäuren entstehen. Selbstverständlich können die zu oxidierenden Verbindungen auch Heteroatome wie Stickstoff, Sauerstoff oder Schwefel in der Kette bzw. dem Ring enthalten. Insbesondere kann das Verfahren gemäss der vorliegenden Erfindung zur Oxidation zyklischer Kohlenwasserstoffe wie Cyclohexen, Cyclohexadien, substituierten ungesättigten C₆-Zyklen, Cyclohepten, Cycloheptadien, substituierten ungesättigten C₇-Zyklen, Cycloocten, Cyclooctadien, substituierten ungesättigten C₈-Zyklen, Cyclononen, Cyclononadien, substituierten ungesättigten C₉-Zyklen, Cyclodecen, Cyclodecadien, substituierten ungesättigten C₁₀-Zyklen, Cycloundecen, Cycloundecadien, substituierten ungesättigten C₁₁-Zyklen, Cyclododecen, Cyclododecadien, Cyclododecatrien, substituierten ungesättigten C₁₂-Zyklen, Cyclopentadecen, Cyclopentadecadien, Cyclopentadecatrien, substituierten ungesättigten C₁₅-Zyklen, Trivinylcyclohexan oder Trivinylcyclohexen eingesetzt werden.

Durch die erfindungsgemäße allylische Oxidation einer der vorgenannten Verbindungen entsteht ein ein Gemisch mehrerer Verbindungen. So zeigt die Analyse der Oxidationsprodukte mittels der Gaschromatographie eine Vielzahl von Verbindungen. Die Anzahl der Verbindungen sowie die Verbindungen selbst sind allerdings nicht erfindungswesentlich, da die Oxidationsprodukte nur Zwischenprodukte bei der Herstellung der Alkohole, Ketone, Aldehyde bzw. Carbonsäuren darstellen. Die Oxidationsprodukte werden einer Hydrierung zugeführt. Das Hydrierungsprodukt weist überraschenderweise mit hoher Selektivität weniger Verbindungen auf als vor der Hydrierung.

Das Reaktionsgemisch enthält auch einen Radikalstarter, der entweder selbst ein Radikal darstellt oder unter Bildung von Radikalen zerfällt, wie eine Peroxyverbindung oder eine Azoverbindung. Beispiele für solche Verbindungen sind Cumolhydroperoxid, Cyclohexylbenzolhydroperoxid, Cyclododecylbenzolhydroperoxid, Ethylbenzolhydroperoxid, 1,4-Di(2-neodecanoyl-peroxyisopropyl)benzol, Acetylcyclohexansulfonylperoxid, Cumylperoxyneodecanoat, Dicyclohexylperoxydicarbonat, Di(4-tert.-butylcyclohexyl)peroxydicarbonat, Di(2-ethylhexyl)peroxydicarbonat, Dimyristylperoxydicarbonat, Dicetylperoxydicarbonat, tert.-Butylperoxyneodecanoat, tert.-Amylperoxyneodecanoat, tert.-Amylperoxypivalat, tert.Butylperoxypivalat, Diisononanoylperoxid, Didecanoylperoxid, tert.-Butylperoxy-2-ethylhexanoat, tert.-Butylperoxyisononanoat, 2,2'-Di-tert.-Butylperoxybutan, Di-tert.-Butylperoxybenzoat, Di-tert.-Butylperoxid, tert.-Butylhydroperoxid, 3,4-Dimethyl-3,4-diphenylhexan, Dibenzoylperoxid, 1,4-Di-tert-butylperoxycyclohexan, tert.-Butylperoxyethylhexylcarbonat, 1,1-Di-tert.-butylperoxycyclohexan, 2,2'-Azobis(2,4-dimethylvaleronitril, 2,2'-Azobis(4-methoxy-2,4-dimethylvaleronitril 2,2'-Azobis(2-methylpropionitril), 1,1'-Azobis(cyclohexancarbonitril), Cyclohexylhydroperoxid. Selbstverständlich können auch intermediär gebildete Peroxide und insbesondere Azoverbindungen als Radikalstarter verwendet werden.

Bevorzugt wird ein Radikalstarter, der ein an ein sekundäres oder tertiäres Kohlenstoffatom gebundenes Sauerstoffatom enthält, eingesetzt. Besonders bevorzugt wird ein Radikalstarter, der sich vom Endprodukt ableitet und mindestens ein an ein oder tertiäres Kohlenstoffatom gebundenes Sauerstoffatom enthält, eingesetzt. Ganz besonders bevorzugt sind Azoinitiatoren. Der Radikalstarter wird entweder separat zugegeben oder wie oben erwähnt während der Reaktion intermediär erzeugt. Der Radikalstarter kann auch, wenn das Reaktionsgefäß nicht absolut gereinigt werden kann, in geringen Mengen aus vorausgegangenen Reaktionen noch im Reaktionsgefäß vorhanden sein.

Die Konzentration des Radikalstarters im erfindungsgemässen Verfahren ist zu Beginn der Reaktion häufig geringer als die Konzentration des Katalysators. Es ist jedoch zu beachten, dass im Verlauf der Reaktion eine intermediäre Bildung von Radikalstarter erfolgen kann, so dass sich die Konzentration von Radikal startenden Verbindungen im Verlauf der Reaktion erhöhen kann.

Die gebildeten Oxidationsprodukte können prinzipiell als solche isoliert werden, erfindungsgemäss ist die direkte anschliessende Hydrierung die Regel. Diese erfolgt entweder in demselben Reaktionsgefäss oder sie erfolgt als zweite Stufe in einem separaten Reaktionsbehälter.

Das erfindungsgemässe Verfahren kann sowohl batchweise als auch kontinuierlich durchgeführt werden.

Das erfindungsgemässe Verfahren kann unter Verwendung eines sauerstoffhaltigen Gases als Oxidationsmittel durchgeführt werden. Der Anteil des Sauerstoffs in dem Gas kann zwischen 5 bis 100 Vol-% betragen. Bevorzugt wird Luftsauerstoff oder reiner Sauerstoff als Oxidationsmittel verwendet. Es ist in jedem Fall auf eine innige Vermischung der flüssigen und der gasförmigen Phase zu achten. Dies kann z. B. in Rührkesseln durch eine entsprechende Rührgeschwindigkeit oder durch Einbauten und in Rohrreaktoren mit Packungselementen sowie mit Blasensäulen erreicht werden.

Das erfindungsgemässe Verfahren kann sowohl unter Atmosphärendruck als auch unter erhöhtem Druck bis zu 100 bar durchgeführt werden. Bevorzugt wird ein Druck von 1 bar bis 50 bar, besonders bevorzugt wird ein Druck von 1 bar bis 20 bar.

Die Hydrierung wird mit Wasserstoff z. B. in entsprechenden Reaktionsbehältem unter erhöhtem Druck bis zu 100bar, bevorzugt bis zu 50 bar, ganz besonders bevorzugt bis zu 20 bar und in einer speziellen Ausführungsform bis zu 10 bar unter Verwendung geeigneter Katalysatoren wie z. B. dem sog. Ru/C-Katalysator (Engelhard Corp., 101 Wood Av., Iselin, N.J. 08830-0770) ausgeführt. Zur Hydrierung kann es vorteilhaft sein, den Oxidationskatalysators vor der Hydrierung von den Oxidationsprodukten abzutrennen. Das Abtrennen kann auf verschiedene Weise erfolgen, wie z. B. durch Einsatz einer Membran oder durch Zugabe eines Lösemittels, in dem der Katalysator unlöslich ist und anschließende Phasentrennung. Derartige Lösemittel sind beispielsweise chlorierte Kohlenwasserstoffe wie z. B. Tetrachlorkohlenstoff. Selbstverständlich kann nicht nur der genannte Hydrierkatalysator, sondern es kann eine Vielzahl marktgängiger, für diesen Zweck angebotener Katalyatoren verwendet werden. Bevorzugt wird der Ru/C-Katalysator der Fa. Engelhard verwendet. Bezogen auf das Substrat werden davon 10⁻⁵ bis 10² mol %, besonders bevorzugt von 10⁻³ bis 20 mol % und ganz besonders bevorzugt von 10⁻² bis 10 mol % eingesetzt. Die Hydrierung wird bei einer Temperatur von 0 bis 500 °C durchgeführt, bevorzugt sind Temperaturen von 20 bis 300 °C, ganz besonders bevorzugt sind Temperaturen von 40 bis 200 °C.

Die folgenden Beispiele sollen das erfindungsgemäße Verfahren näher erläutern, ohne die Erfindung auf diese Verfahren zu beschränken.

### Abkürzungen:

PhCN = Benzonitril
NHPI = N-Hydroxyphthalimid
V-65 = 2,2'-Azobis (2,4-dimethylvaleronitril)
V-70 = 2,2'-Azobis (4-methoxy-2,4-dimethylvaleronitril)
DCP = Dibenzoylperoxid
CDT = Cyclododecatrien

### Beispiel 1 (erfindungsgemäss)

2 mmol CDT, 5 ml Aceton, 8 mol % NHPI, 1 mol % V-70 werden in einem Rundkolben mit aufgesetztem Rückflusskühler bei einer Temperatur von 50 °C 5 Stunden lang gerührt. Dabei wird unter 1 bar Druck Sauerstoff durch die Mischung geleitet. Nach erfolgter Hydrierung unter Verwendung von 5 mol % des Katalysators Ru/C in einem Laborautoklaven bei 10 bar Druck und einer Temperatur von 100 °C werden bei einem Umsatz von 15 % CDT in einer Selektivität von 98 % Cyclododecanon und Cyclododecanol etwa im Verhältnis 1 : 1 erhalten.

### Beispiel 2 (erfindungsgemäss)

2 mmol CDT, 5 ml PhCN, 4 mol % NHPI, 1 mol % V-65 werden in einem Rundkolben mit aufgesetztem Rückflusskühler bei einer Temperatur von 70 °C 24 Stunden lang gerührt. Dabei wird unter 1 bar Druck Sauerstoff durch die Mischung geleitet. Nach erfolgter Hydrierung mit 5 mol % Katalysator Ru/C bei 100 °C in einem Laborautoklaven bei 15 bar Druck werden bei einem Umsatz von 55 % CDT in einer Selektivität von 81 % Cyclododecanon und Cyclododecanol etwa im Verhältnis 1:1 erhalten.

### Beispiel 3 (erfindungsgemäss, in Masse)

2 mmol CDT, 4 ml CDT (als Lösemittel), 2 mol % NHPI, 1 mol % DBP werden in einem Rundkolben mit aufgesetztem Rückflusskühler bei einer Temperatur von 50 °C 6 Stunden lang gerührt. Dabei wird unter 1 bar Druck Sauerstoff durch die Mischung geleitet. Nach erfolgter Hydrierung mit 5 mol % Katalysator Ru/C bei 100 °C in einem Laborautoklaven bei 8 bar Druck werden bei einem Umsatz von 22 % CDT (insgesamt, d. h. einschliesslich CDT als Lösemittel) in einer Selektivität von 79 % Cyclododecanon und Cyclododecanol etwa im Verhältnis 1 : 1 erhalten.

### Beispiel 4 (nicht erfindungsgemäss, ohne NHPI)

2 mmol CDT, 5 ml Aceton und 1 mol % V-70 werden in einem Rundkolben mit aufgesetztem Rückflusskühler bei einer Temperatur von 50 °C 5 Stunden lang gerührt. Dabei wird unter 1 bar Druck Sauerstoff durch die Mischung geleitet. Nach erfolgter Hydrierung mit 5 mol % Katalysator Ru/C bei 100 °C in einem Laborautoklaven bei 8 bar Druck werden bei einem Umsatz von 4 % CDT in einer Selektivität von 12 % ein Cyclododecanon-/Cyclododecanol-Gemisch 1 : 1 erhalten.

### Beispiel 5 (nicht erfindungsgemäss, mit Kobaltkatalysator)

2 mmol CDT, 5 ml PhCN, 4 mol % NHPI und 4 mol % Co(II)-Acetat werden in einem Rundkolben mit aufgesetztem Rückflusskühler bei einer Temperatur von 70 °C 24 Stunden lang gerührt. Dabei wird unter 1 bar Druck Sauerstoff durch die Mischung geleitet. Nach erfolgter Hydrierung mit 5 mol % Katalysator Ru/C bei 100 °C in einem Laborautoklaven bei 8 bar Druck werden bei einem Umsatz von 67 % CDT in einer Selektivität von 6 % ein Cyclododecanon-/Cyclododecanol-Gemisch erhalten.

## Patentansprüche

1. Verfahren zur Herstellung von gesättigten Alkoholen, Ketonen, Aldehyden und Carbonsäuren, wobei als Ausgangsverbindung ein ungesättigter Kohlenwasserstoff eingesetzt wird, der mit einem sauerstoffhaltigen Gas unter Verwendung eines Katalysators und bei Anwesenheit eines Radikalstarters zunächst oxidiert wird, und als Katalysator eine Verbindung der Formel **I** 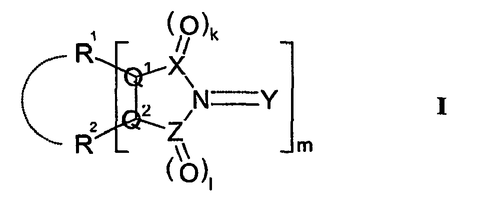 mit R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können,
Q¹, Q² = gleich oder ungleich, C, CH oder N,
X, Z = C, S oder CH₂,
Y = O oder OH,
k = 0, 1 oder 2,
l = 0, 1 oder 2 und
m = 1 bis 100
eingesetzt wird,
**dadurch gekennzeichnet,**
**dass** die Oxidationsprodukte durch eine anschliessende Hydrierung reduziert werden und gesättigte Alkohole, Ketone, Aldehyde und/oder Carbonsäure gebildet werden.

2. Verfahren gemäss Anspruch 1,
**dadurch gekennzeichnet,**
**dass** als Katalysator eine Verbindung der Formel **II** 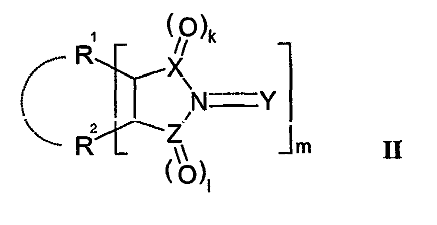 mit R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können,
X, Z = C, S oder CH₂,
Y = O oder OH,
k = 0, 1 oder 2,
l = 0, 1 oder 2 und
m = 1 bis 3 eingesetzt wird.

3. Verfahren nach einem der Ansprüche 1 oder 2,
**dadurch gekennzeichnet,**
**dass** als Katalysator eine Verbindung der Formel **III** 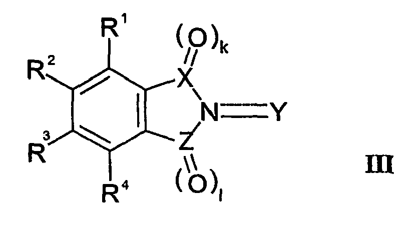 mit R¹, R², R³ und R⁴ = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹, R², R³ und R⁴ identische oder unterschiedliche Reste bezeichnen können,
X, Z = C, S oder CH₂,
Y = O oder OH,
k = 0, 1 oder 2 und
l = 0, 1 oder 2
eingesetzt wird.

4. Verfahren nach Anspruch 1,
**dadurch gekennzeichnet,**
**dass** ein Hydantoinderivat der Formel IV 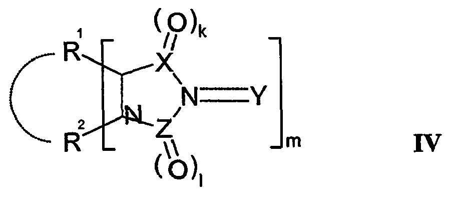 mit R¹, R² = H, aliphatischer oder aromatischer Alkoxyrest, Carboxylrest, Alkoxycarbonylrest oder Kohlenwasserstoffrest, jeweils mit 1 bis 20 Kohlenstoffatomen, SO₃H, NH₂, OH, F, Cl, Br, I und/oder NO₂, wobei R¹ und R² identische oder unterschiedliche Reste bezeichnen oder R¹ und R² über eine kovalente Bindung miteinander verknüpft sein können,
X, Z = C, S oder CH₂,
Y = O oder OH,
k = 0, 1 oder 2,
l = 0, 1 oder 2 und
m = 1 bis 3 als Katalysator eingesetzt wird.

5. Verfahren nach zumindest einem der Ansprüche 1 bis 4,
**dadurch gekennzeichnet,**
**dass** als ungesättigter Kohlenwasserstoff ein lineares oder verzweigtes Alken, Alkin, Dien oder Trien mit einer Kohlenstoffzahl von 3 bis 25 eingesetzt wird.

6. Verfahren nach zumindest einem der Ansprüche 1 bis 4,
**dadurch gekennzeichnet,**
**dass** als ungesättigter Kohlenwasserstoff ein zyklisches Alken, Dien oder Trien mit 5 bis 25 Ringatomen eingesetzt wird.

7. Verfahren gemäß Anspruch 6
**dadurch gekennzeichnet,**
**dass** als zu oxidierender zyklischer Kohlenwasserstoff zumindest eine Verbindung ausgewählt aus Cyclohexen, Cyclohexadien, substituierten ungesättigten C₆-Zyklen, Cyclohepten, Cycloheptadien, substituierten ungesättigten C₇-Zyklen, , Cycloocten, Cyclooctadien, substituierten ungesättigten C₈-Zyklen, Cyclononen, Cyclononadien, substituierten ungesättigten C₉-Zyklen, Cyclodecen, Cyclodecadien, substituierten ungesättigten C₁₀-Zyklen, Cycloundecen, Cycloundecadien, substituierten ungesättigten C₁₁-Zyklen, Cyclododecen, Cyclododecadien, Cyclododecatrien, substituierten ungesättigten C₁₂-Zyklen, Cyclopentadecen, Cycolopentadecadien, Cyclopentadecatrien, substituierten ungesättigten C₁₅-Zyklen, Trivinylcyclohexan oder Trivinylcyclohexen eingesetzt wird.

8. Verfahren nach einem der Ansprüche 1 bis 7,
**dadurch gekennzeichnet,**
**dass** als Radikalstarter eine Peroxyverbindung oder Azoverbindung eingesetzt wird.

9. Verfahren nach einem der Ansprüche 1 bis 8,
**dadurch gekennzeichnet,**
**dass** die katalytische Oxidation bei einer Temperatur von 0 bis 500 °C durchgeführt wird.

10. Verfahren nach einem der Ansprüche 1 bis 9,
**dadurch gekennzeichnet,**
**dass** als Oxidationsmittel ein Gas, welches 5 bis 100 Vol.-% Sauerstoff aufweist, verwendet wird.

11. Verfahren nach einem der Ansprüche 1 bis 10,
**dadurch gekennzeichnet,**
**dass** die katalytische Oxidation unter einem Druck von 1 bis 100 bar durchgeführt wird.

## Claims

1. A process for preparing a saturated alcohol, ketone, aldehyde or carboxylic acid, an unsaturated hydrocarbon which is first oxidized with an oxygen-containing gas, using a catalyst and in the presence of a free-radical initiator being used as the starting compound, and a compound of the formula **I** 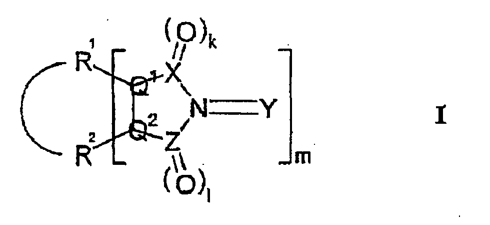 being used as the catalyst, where R¹, R² = H, aliphatic or aromatic alkoxy radical, carboxyl radical, alkoxycarbonyl radical or hydrocarbon radical, each of which has 1 to 20 carbon atoms, SO₃H, NH₂, OH, F, Cl, Br, I and/or NO₂, R¹ and R² being identical or different radicals, or it being possible for R¹ and R² to be linked to one another via a covalent bond,
Q₁, Q₂ = C, CH or N, identical or different,
X, Z = C, S or CH₂,
Y = O or OH,
k = 0, 1 or 2,
l = 0, 1 or 2 and
m = 1 to 100,
**characterized in that** the oxidation product is reduced by a subsequent hydrogenation and a saturated alcohol, ketone, aldehyde and/or carboxylic acid is formed.

2. A process according to claim 1, **characterized in that** a compound of the formula **II** 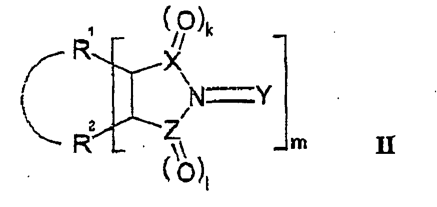 is used as the catalyst, where R¹, R² = H, aliphatic or aromatic alkoxy radical, carboxyl radical, alkoxycarbonyl radical or hydrocarbon radical, each of which has 1 to 20 carbon atoms, SO₃H, NH₂, OH, F, Cl, Br, I and/or NO₂, R¹ and R² being identical or different radicals, or it being possible for R¹ and R² to be linked to one another via a covalent bond,
X, Z = C, S or CH₂,
Y = 0 or OH,
k = 0, 1 or 2,
l = 0, 1 or 2 and
m = 1 to 3.

3. A process according to either one of claims 1 and 2, **characterized in that** a compound of the formula **III** 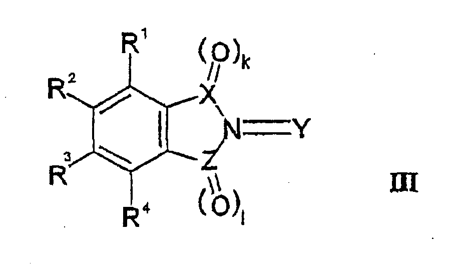 is used as the catalyst, where R¹, R², R³ and R⁴ = H, aliphatic or aromatic alkoxy radical, carboxyl radical, alkoxycarbonyl radical or hydrocarbon radical, each of which has 1 to 20 carbon atoms, SO₃H, NH₂, OH, F, Cl, Br, I and/or NO₂, it being possible for R¹, R², R³ and R⁴ to be identical or different radicals,
X, Z = C, S or CH₂,
Y = O or OH,
k = 0, 1 or 2 and
l = 0, 1 or 2.

4. A process according to claim 1, **characterized in that** a hydantoin derivative of the formula **IV** 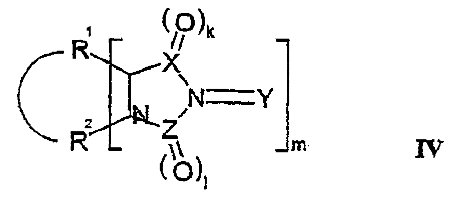 where R¹, R² = H, aliphatic or aromatic alkoxy radical, carboxyl radical, alkoxycarbonyl radical or hydrocarbon radical, each of which has 1 to 20 carbon atoms, SO₃H, NH₂, OH, F, Cl, Br, I and/or NO₂, R¹ and R² being identical or different radicals, or it being possible for R¹ and R² to be linked to one another via a covalent bond,
X, Z = C, S or CH₂,
Y = 0 or OH,
k = 0, 1 or 2,
**l** = 0, 1 or 2 and
m = 1 to 3 is used as catalyst.

5. A process according to at least one of claims 1 to 4, **characterized in that** an unbranched or branched alkene, alkyne, diene or triene having a carbon number from 3 to 25 is used as the unsaturated hydrocarbon.

6. A process according to at least one of claims 1 to 4, **characterized in that** a cyclic alkene, diene or triene having 5 to 25 ring atoms is used as the unsaturated hydrocarbon.

7. A process according to claim 6, **characterized in that** at least one compound selected from the group consisting of cyclohexene, cyclohexadiene, substituted unsaturated cyclic C₆ compounds, cycloheptene, cycloheptadiene, substituted unsaturated cyclic C₇ compounds, cyclooctene, cyclooctadiene, substituted unsaturated cyclic C₈ compounds, cyclononene, cyclononadiene, substituted unsaturated cyclic C₉ compounds, cyclodecene, cyclodecadiene, substituted unsaturated cyclic C₁₀ compounds, cycloundecene, cycloundecadiene, substituted unsaturated cyclic C₁₁ compounds, cyclododecene, cyclododecadiene, cyclododecatriene, substituted unsaturated cyclic C₁₂ compounds, cyclopentadecene, cyclopentadecadiene, cyclopentadecatriene, substituted unsaturated cyclic C₁₅ compounds, trivinylcyclohexane or trivinylcyclohexene is used as the cyclic hydrocarbon to be oxidized.

8. A process according to any one of claims 1 to 7, **characterized in that** a peroxy compound or azo compound is used as the free-radical initiator.

9. A process according to any one of claims 1 to 8, **characterized in that** the catalytic oxidation is carried out at a temperature of 0 to 500°C.

10. A process according to any one of claims 1 to 9, **characterized in that** a gas which comprises 5 to 100% by volume of oxygen is used as the oxidizing agent.

11. A process according to any one of claims 1 to 10, **characterized in that** the catalytic oxidation is carried out at a pressure of 1 to 100 bar.

## Revendications

1. Procédé de production d'alcools, cétones, aldéhydes et acides carboxyliques saturés, en utilisant comme composé de départ un hydrocarbure insaturé, qui est d'abord oxydé avec un gaz contenant de l'oxygène avec utilisation d'un catalyseur et en présence d'un initiateur radicalaire, et avec comme catalyseur un composé répondant à la formule 1 : 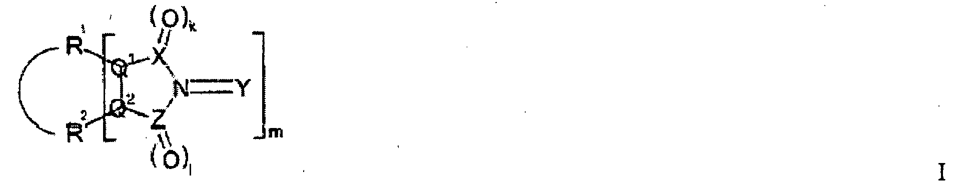 dans laquelle R¹ et R² représentent H, un radical alcoxy, carboxyle, alcoxycarbonyle ou hydrocarbure, aliphatique ou aromatique, comportant à chaque fois 1 à 20 atomes de carbone, SO₃ H, NH₂, OH, F, Cl, Br, I et/ou NO₂, R¹ et R² désignant des radicaux identiques ou différents, ou R¹ et R² pouvant être reliés l'un à l'autre au moyen d'une liaison covalente,
Q¹, Q² sont identiques ou différents et représentent C, CH ou N,
X, Z représentent C, S ou CH₂,
Y représente O ou OH,
k a pour valeur 0, 1 ou 2,
l a pour valeur 0, 1 ou 2, et
m a pour valeur 1 à 100,
**caractérisé en ce qu'**
on réduit les produits d'oxydation au moyen d'une hydrogénation subséquente, et on forme des alcools, cétones, aldéhydes et/ou acides carboxyliques saturés.

2. Procédé selon la revendication 1,
**caractérisé en ce qu'**
on utilise comme catalyseur un composé répondant à la formule II 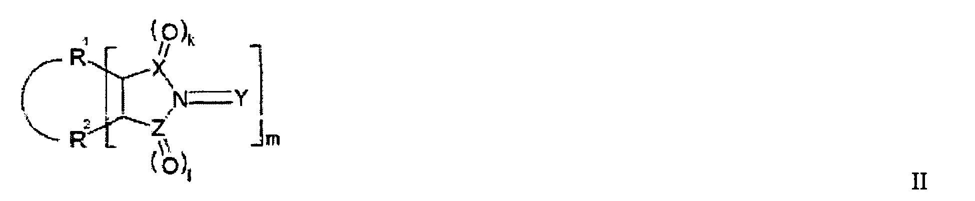 dans laquelle R¹, R² représentent H, un radical alcoxy, carboxyle, alcoxycarbonyle ou hydrocarbure, aliphatique ou aromatique, comportant à chaque fois 1 à 20 atomes de carbone, SO₃ H, NH₂, OH, F, Cl, Br, I et/ou NO₂, R¹ et R² désignant des radicaux identiques ou différentes, ou R¹ et R² pouvant être reliés l'un à l'autre au moyen d'une liaison covalente,
X, Z représentent C, S ou CH2,
Y représente O ou OH,
k a pour valeur 0, 1 ou 2,
l a pour valeur 0, 1 ou 2, et
m a pour valeur 1 à 3.

3. Procédé selon l'une ou l'autre des revendications 1 ou 2,
**caractérisé en ce qu'**
on utilise comme catalyseur un composé répondant à la formule III 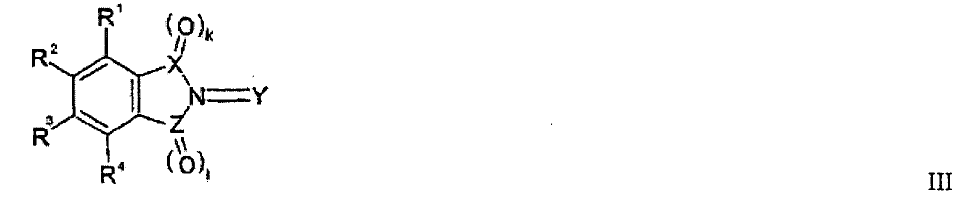 dans laquelle R¹, R², R³ et R⁴ représentent H, un radical alcoxy, carboxyle, alcoxycarbonyle ou hydrocarbure, aliphatique ou aromatique, comportant à chaque fois 1 à 20 atomes de carbone, SO₃ H, NH₂, OH, F, Cl, Br, I et/ou NO₂, et R¹, R², R³ et R⁴ pouvant désigner des radicaux identiques ou différents,
X, Z représentent C, S ou CH₂,
Y représente O ou OH,
k a pour valeur 0, 1 ou 2 et
l a pour valeur 0, 1 ou 2.

4. Procédé selon la revendication 1,
**caractérisé en ce qu'**
on utilise, comme catalyseur, un dérivé d'hydantonine répondant à la formule IV, 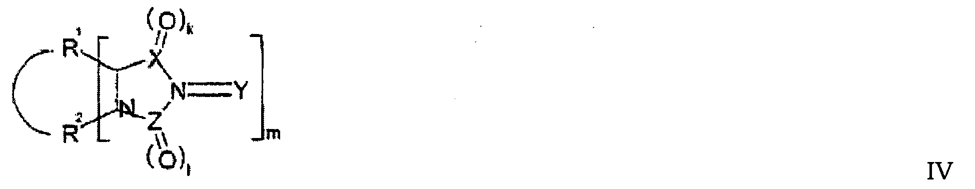 dans laquelle R¹, R² représentent H, un radical alcoxy, carboxyle, alcoxycarbonyle ou hydrocarbure, aliphatique ou aromatique, comportant à chaque fois 1 à 20 atomes de carbone, SO₃ H, NH₂, OH, F, Cl, Br, I et/ou NO₂, et R¹, R² désignant des radicaux identiques ou différents, ou R¹, R² pouvant être reliés l'un à l'autre au moyen d'une liaison covalente,
X, Z représentent C, S ou CH₂,
Y représente O ou OH,
k a pour valeur 0, 1 ou 2,
l a pour valeur 0, 1 ou 2, et
m a pour valeur 1 à 3.

5. Procédé selon au moins une des revendications 1 à 4,
**caractérisé en ce qu'**
on utilise, comme hydrocarbure insaturé, un alcène, un alcyne, un diène ou un triène linéaire ou ramifié présentant un nombre d'atomes de carbone de 3 à 25.

6. Procédé selon au moins une des revendications 1 à 4,
**caractérisé en ce qu'**
on utilise comme hydrocarbure insaturé un alcène, un diène ou un triène cyclique comportant 5 à 25 atomes de cycles.

7. Procédé selon la revendication 6,
**caractérisé en ce qu'**
on utilise, comme hydrocarbure cyclique à oxyder, au moins un composé choisi parmi le cyclohexène, le cyclohexadiène, des composés cycliques en C₆ insaturés substitués, le cycloheptène, le cycloheptadiène, des composés cycliques en C₇ insaturés substitués, le cyclooctène, le cyclooctadiène, des composés cycliques en C₈ insaturés substitués, le cyclononène, le cyclononadiène, des composés cycliques C₉ insaturés substitués, le cyclodécène, le cyclodécadiène, des composés cycliques en C₁₀ insaturés substitués, le cycloundécène, le cycloundécadiène, des composés cycliques en C₁₁ insaturés substitués, le cyclododécène, le cyclododécadiène, le cyclododécatriène, des composés cycliques en C₁₂ insaturés substitués, le cyclopentadécène, le cyclopentadécadiène, le cyclopentadécatriène, des composés cycliques en C₁₅ insaturés substitués, le trivinylcyclohexane ou le trivinylcyclohexène.

8. Procédé selon une quelconque des revendications 1 à 7,
**caractérisé en ce qu'**
on utilise, comme initiateur radicalaire, un composé peroxy ou un composé azo.

9. Procédé selon une quelconque des revendications 1 à 8,
**caractérisé en ce que**
l'oxydation catalytique est effectuée à une température de 0 à 500°C.

10. Procédé selon une quelconque des revendications 1 à 9,
**caractérisé en ce qu'**
on utilise comme agent d'oxydation un gaz qui présente une teneur de 5 à 100 % en volume d'oxygène.

11. Procédé selon une quelconque des revendications 1 à 10,
**caractérisé en ce que**
l'oxydation catalytique est effectuée sous une pression de 1 à 100 bars.
